# EUROPÄISCHE PATENTSCHRIFT

(11) **EP 1 030 827 B1**
(45) Veröffentlichungstag und Bekanntmachung des Hinweises auf die Patenterteilung: **27.03.2002**
(21) Anmeldenummer: 98955468.8
(22) Anmeldetag: 16.10.1998
(51) Int. Cl.: C07C 29/149, C07C 31/20

(54) **VERFAHREN ZUR HERSTELLUNG VON 1,6-HEXANDIOL UND 6-HYDROXYCAPRONSÄURE BZW. DEREN ESTERN**
METHOD FOR PRODUCING 1,6-HEXANEDIOL AND 6-HYDROXYCAPROIC ACID OR THEIR ESTERS
PROCEDE DE PRODUCTION DE 1,6 HEXANEDIOL ET D'ACIDE 6-HYDROXYCAPROIQUE OU DE SES ESTERS

(30) Priorität: 14.11.1997 DE 19750532
(43) Veröffentlichungstag der Anmeldung: 30.08.2000
(73) Patentinhaber: BASF AKTIENGESELLSCHAFT, 67056 Ludwigshafen (DE)
(72) Erfinder: FISCHER, Rolf, Hartmuth, D-69121 Heidelberg (DE); PINKOS, Rolf, D-67098 Bad Dürkheim (DE); STEIN, Frank, D-67098 Bad Dürkheim (DE)
(86) Internationale Anmeldenummer: EP9806574
(87) Internationale Veröffentlichungsnummer: WO9925672

(56) Entgegenhaltungen:
- EP-A- 0 722 923
- EP-A- 0 724 908

## Beschreibung

Die Erfindung betrifft ein Verfahren zur verbesserten Herstellung von 1,6-Hexandiol und 6-Hydroxycapronsäure bzw. deren Estern ausgehend von Adipinsäure bzw. deren Mono- und Diestern oder Kohlenwasserstoffströmen, die Adipinsäure bzw. deren Mono- und Diester und 6-Hydroxycapronsäure bzw. deren Ester enthalten, durch katalytische Hydrierung der Säuren und/oder der Ester und Rückführung der nach Destillation des Hydrierprodukts als Sumpf anfallenden dimeren und oligomeren Verbindungen.

Aus US 2 066 533 ist bekannt, Dicarbonsäuren und deren Ester katalytisch partiell zu den entsprechenden Hydroxycarbonsäuren bzw. deren Lactonen zu hydrieren, ohne wesentliche Anteile an Diolen herzustellen.

Aus EP 724 908 A1 ist bekannt, an (modifizierten) Raney-Edelmetallkatalysatoren Adipinsäure oder deren Ester zu 1,6-Hexandiol und 6-Hydroxycapronsäure oder deren Ester zu hydrieren.

Aus JP 49 132 003 ist bekannt, Adipinsäure an MO/CO/SiO₂-Katalysatoren zu 1,6-Hexandiol und 6-Hydroxycapronsäure zu hydrieren.

Die vorgenannten Verfahren haben den Nachteil, daß die während der Hydrierung entstehenden Produktgemische, d.h. Alkohole und Carbonsäuren, dimere und oligomere Ester enthalten.

Beispielhaft seien Ester aus Adipinsäure mit Hexandiol und Hydroxycarbonsäure sowie Ester aus Hydroxycapronsäure und Hexandiol genannt (diese Ester werden im folgenden als Dimere bezeichnet). Diese Ester sind für eine weitere Anwendung in Richtung 6-Hydroxycapronsäure(ester) bzw. 1,6-Hexandiol verloren bzw. müssen nach destillativer Abtrennung der Wertprodukte in einem weiteren Verfahrensschritt wie z.B. Hydrolyse mit Wasser, die als Gleichgewichtsreaktion keine vollständigen Umsätze ergibt, gespalten werden. Damit sind die oben beschriebenen Verfahren nur bedingt wirtschaftlich und es bestand die Aufgabe, diesen Nachteil des Standes der Technik zu überwinden.

Es wurde nun überraschend gefunden, daß man die Gesamtausbeute an 6-Hydroxycapronsäure bzw. deren Ester und 1,6-Hexandiol mit einem Verfahren zur Herstellung von 1,6-Hexandiol und 6-Hydroxycapronsäure oder deren Estern durch katalytische Hydrierung von Adipinsäure, Adipinsäuremonoestern oder Adipinsäurediestern oder Eduktströmen, die Adipinsäure oder deren Ester als wesentliche Bestandteile enthalten, deutlich steigern kann, wenn man das bei der Destillation des Hydrieraustrags nach Abtrennung des Hexandiols und der Hydroxycapronsäure bzw. deren Ester erhaltene Sumpfprodukt, das im wesentlichen oligomere Ester der 6-Hydroxycapronsäure enthält, in die Hydrierung zurückführt und das so erhaltene Gemisch aus Edukt und Rückführstrom bei Temperaturen von 100 bis 300°C und Drücken von 10 bis 300 bar in der Flüssigphase und einem Molverhältnis von zu hydrierenden Carboxylgruppen zu Wasserstoff im Reaktor von 1 : 5 bis 1 : 100 an Hydrierkatalysatoren umsetzt.

Es war überraschend, daß sich die rückgeführten C₆-Dimeren und C₆-Oligomeren unter den Reaktionsbedingungen der Hydrierung der monomeren Säuren oder deren Ester ohne unmittelbare Aufpegelung von den Dimeren, Oligomeren und Nebenprodukten zu 1,6-Hexandiol und 6-Hydroxycapronsäure bzw. deren Ester umsetzen lassen und daß sich die Selektivität der Reaktion nicht verschlechtert. Weiterhin war überraschend, daß die Katalysatorstandzeit nicht durch die Rückführung beeinträchtigt wird, denn es war anzunehmen, daß sich dimere und oligomere Verbindungen teilweise auf dem Katalysator ablagern und diesen dadurch in seiner Aktivität und Selektivität beeinträchtigen.

Die Ester der Adipinsäure bzw. 6-Hydroxycapronsäure enthalten als Alkoholkomponente bevorzugt Methanol, Ethanol, n-Propanol, i-Propanol, n-Butanol, i-Butanol und n-Pentanol. Ein spezieller Ester der Hydroxycapronsäure ist der innere Ester Caprolacton. Als Edukt für die Hydrierung dient Adipinsäure bzw. deren Mono- und/oder Diester. In diesem Edukt können noch weitere C₆-Verbindungen wie z.B. 6-Hydroxycapronsäure bzw. deren Ester enthalten sein. Möglich sind auch noch weitere, nicht C₆-Verbindungen, ohne daß sie das erfindungsgemäße Verfahren beeinträchtigen, z.B. Säuren wie Glutarsäure oder Bernsteinsäure bzw. deren Ester. Solche Gemische sind beispielsweise in DE-A 19 607 953 beschrieben. Für das erfindungsgemäße Verfahren sind z.B. aus dem Patentbeispiel l.c. der Strom aus Stufe 4 oder der Kopfstrom aus Stufe 12 einsetzbar.

Die Hydrierung wird in der Flüssigphase durchgeführt. Als Hydrierkatalysatoren finden im erfindungsgemäßen Verfahren im allgemeinen heterogene, aber auch homogene, zur Hydrierung von Carbonylgruppen geeignete Katalysatoren Verwendung. Sie können sowohl fest angeordnet, als auch mobil, z.B. in einem Wirbelbettreaktor, eingesetzt werden. Beispiele hierfür sind z.B. in Houben-Weyl, Methoden der Organischen Chemie, Band IV/1c, S. 16 bis 26 beschrieben.

Von den erfindungsgemäß zu verwendenden Hydrierkatalysatoren sind solche bevorzugt, die ein oder mehrere Elemente der Gruppe Ib, VIb, VIIb und VIIIb, sowie IIIa, IVa und Va des Periodensystems der Elemente, insbesondere Kupfer, Chrom, Rhenium, Kobalt, Rhodium, Nickel, Palladium, Eisen, Platin, Indium, Zinn und/oder Antimon enthalten. Besonders bevorzugt sind Katalysatoren, die Kupfer, Kobalt und/oder Rhenium enthalten.

Die im erfindungsgemäßen Verfahren eingesetzten Katalysatoren können z.B. sogenannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, z.B. schwerlösliche Hydroxide, Oxidhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C in die entsprechenden Oxide, Mischoxide und/oder gemischtvalentigen Oxide umwandelt, welche durch eine Behandlung mit Wasserstoff oder mit Wasserstoff enthaltenden Gasen bei in der Regel 50 bis 700°C, insbesondere 100 bis 400°C zu den betreffenden Metallen und/oder oxidischen Verbindungen niederer Oxidationsstufe reduziert und in die eigentliche, katalytisch aktive Form überführt werden.. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden. Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den oben genannten Fällungskatalysatoren, welche außer den katalytisch aktiven Komponenten noch zusätzlich ein Trägermaterial enthalten, eignen sich für das erfindungsgemäße Verfahren im allgemeinen solche Trägermaterialien, bei denen die katalytisch-hydrierend wirkende Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diesen Trägermaterialien z.B. durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder thermisch leicht zersetzbaren Komplexverbindungen, z.B. carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte diese Trägerkatalysatoren an katalytisch aktiven Metallen zu höheren Raum-Zeit-Umsätzen führen können als niedrigere Gehalte. Im allgemeinen werden Trägerkatalysatoren verwendet, deren Gehalt an katalytisch aktiven Metallen 0,1 bis 90 Gew.-%, vorzugsweise 0,5 bis 40 Gew.-%, bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-A 2 519 817, EP-A 1 477 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen, bevorzugt werden diese Katalysatoren jedoch vor ihrer Verwendung separat aktiviert.

Als Trägermaterialien können im allgemeinen die Oxide des Aluminiums und Titans, Zirkoniumdioxid, Siliciumdioxid, Tonerden, z.B. Montmorillonite, Silikate wie Magnesium- oder Aluminiumsilikate, Zeolithe wie ZSM-5 oder ZSM-10-Zeolithe, sowie Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren zu verwendenden Katalysatoren dienen. Als für im erfindungsgemäßen Verfahren einsetzbaren Heterogenkatalysatoren seien die folgenden beispielhaft genannt:

Kobalt auf Aktivkohle, Kobalt auf Siliciumdioxid, Kobalt auf Aluminiumoxid, Rhenium auf Aktivkohle, Rhenium auf Siliciumdioxid, Rhenium/Zinn auf Aktivkohle, Rhenium/Platin auf Aktivkohle, Kupfer auf Aktivkohle, Kupfer/Siliciumdioxid, Kupfer/Aluminiumoxid, Kupferchromit, Bariumkupferchromit, Kupfer/Aluminiumoxid/Manganoxid, Kupfer/Aluminiumoxid/Zinkoxid sowie die Katalysatoren gemäß DE-A 3 932 332, US-A 3 449 445, DP-A 44 444, EP-A 147 219, DE-A 3 904 083, DE-A 2 321 101, EP-A 415 202, DE-A 2 366 264, EP 0 552 463 und EP-A 100 406.

Besonders bevorzugte Katalysatoren enthalten mindestens eines der Metalle Kupfer, Kobalt oder Rhenium.

Das erfindungsgemäße Verfahren kann vorteilhaft kontinuierlich ausgeübt werden. Dabei können beispielsweise Rohrreaktoren eingesetzt werden, in denen der Katalysator vorteilhaft in Form eines Festbetts angeordnet ist.

Das molare Verhältnis von zu hydrierender Gruppe, d.h. Carboxylgruppe entweder als Säuregruppe oder Estergruppe zu Wasserstoff im Reaktor liegt erfindungsgemäß zwischen 1 : 5 und 1 : 100, vorzugsweise zwischen 1 : 7 und 1 : 70. Der notwendige Reaktionsdruck liegt über 10 bar, bevorzugt sind 100 - 300 bar. Besonders bevorzugt sind 150 - 300 bar. Die Reaktionstemperaturen liegen im Bereich von 100 - 300°C. Bevorzugt sind 130 - 270°C, besonders bevorzugt 160 - 240°C. Pro Volumenteil Katalysator kann zwischen 0,05 kg und 5 kg, bevorzugt 0,1 und 3 kg, besonders bevorzugt 0,2 und 1,5 kg Edukt pro Stunde über den Hydrierkatalysator geleitet werden (= Katalysatorbelastung).

Ein Lösungsmittel ist nicht notwendig, kann aber verwendet werden. Beispielsweise kommen Wasser oder der Alkohol der verwendeten Ester wie Methanol, Ethanol usw. in Betracht. Wasser wird bevorzugt bei Verwendung der freien Säure als Lösungsmittel eingesetzt.

Das Verhältnis der Wertprodukte zueinander kann in weiten Bereichen variierbar sein. Bei geringeren Umsätzen dominiert 6-Hydroxycapronsäure bzw. deren Ester. Wirtschaftlich und damit bevorzugt sind molare Verhältnisse von 1,6-Hexandiol zu Hydroxycapronsäure oder deren Ester von 1 zu 5 bis 100 zu 1, bevorzugt 1 zu 2 bis 100 zu 1, besonders bevorzugt 1 zu 1 bis 20 zu 1.

Das Verhältnis kann z.B. durch Wahl der Temperatur, Druck, Katalysatorbelastung oder Verweilzeit beeinflußt werden. Dabei gilt, je geringer Temperatur, Druck und Verweilzeit bzw. je höher die Katalysatorbelastung, desto höher ist der Anteil an Hydroxycapronsäure bzw. deren Ester.

Die Wertprodukte 1,6-Hexandiol und 6-Hydroxycapronsäure bzw. deren Ester werden in an sich bekannter Weise z.B. destillativ bei vermindertem Druck, beispielsweise bei 10 - 500 mbar, bevorzugt 15 - 100 mbar und Sumpftemperaturen von 100 - 250°C, bevorzugt 120 - 220°C gewonnen. Dabei fällt das in die Hydrierung rückführbare Gemisch, das als wesentlichen Bestandteil u.a. mehr als 50 Gew.-%, bevorzugt mehr als 60 Gew.-% und insbesondere mehr als 70 Gew.-% oligomere 6-Hydroxycapronsäureester enthält, als Sumpfprodukt der Destillationskolonne an. Es ist sinnvoll, die Verweilzeit des Gemisches im Sumpf der Destillationskolonne, sofern diese kontinuierlich betrieben wird, insbesondere bei Sumpftemperaturen über 150°C und molaren Anteilen von Hydroxycapronsäure bzw. deren Ester zu Hexandiol im Hydrieraustrag über 1 zu 5 möglichst kurz zu halten, beispielsweise unter 2 Stunden, bevorzugt unter einer Stunde, besonders bevorzugt unter 0,5 Stunden, um zu vermeiden, daß sich im Sumpf hochmolekulare Ester bilden, die die Rückführung erschweren, da sie hohe Schmelzpunkte aufweisen, die über 200°C betragen können.

Die nicht umgesetzte Adipinsäure bzw. deren Ester kann selbstverständlich ebenfalls in die Hydrierung zurückgeführt werden.

Das rückgeführte Sumpfprodukt kann absatzweise, bei Durchführung im technischen Maßstab bevorzugt kontinuierlich rückgeführt werden. Dabei kann der rückgeführte Produktstrom vor dem Reaktor in den Frischzulauf eingemischt, oder als zweiter Zulauf direkt in den Reaktor eingespeist werden. Wird die Hydrierung beispielsweise mit Haupt- und Nachreaktor betrieben, kann der Rückführstrom nicht nur in den Hauptreaktor eingebracht werden, sondern auch in den Nachreaktor. Obgleich in der Regel keine Aufpegelung der Dimeren und Oligomeren und gegebenenfalls von Nebenprodukten eintritt kann es notwendig werden, einen geringen Teil des Sumpfes der Produktdestillation auszuschleusen. Dazu geht man im allgemeinen bei der absatzweisen Fahrweise so vor, daß man mehrfach den gesamten Destillationssumpf zurückführt bis eine Aufpegelung von unerwünschten Produkten eingetreten ist und dann in entsprechenden Zeitabständen einen Teil oder eine gesamte Charge eines Sumpfes ausschleust. Bei kontinuierlicher Durchführung der Verfahren wird zumindest die überwiegende Menge des Destillationssumpfes zurückgeführt und kontinuierlich, soweit erforderlich eine geringere Menge des Sumpfes ausgeschleust.

1,6-Hexandiol stellt einen gesuchten Monomerbaustein dar, der überwiegend auf dem Polyester- und Polyurethansektor eingesetzt wird. 6-Hydroxycapronsäure bzw. deren Ester sind Zwischenprodukte zur Herstellung von Caprolacton, aus dem Polycaprolactone gewonnen werden. Das erfindungsgemäße Verfahren wird anhand der nachfolgenden Beispiele näher beschrieben. Die angegebenen Analysenwerte wurden gaschromatographisch mit inneren Standard bestimmt und sind Gewichts-%.

### Beispiel 1:

In einem 25 ml Rohrreaktor wurden 25 ml Cu-Katalysator der Firma Süd-Chemie mit der Bezeichnung T 4489 gefüllt, der zuvor im Wasserstoffstrom aktiviert worden war. Der Reaktor wurde auf einen Druck von 220 bar und eine Temperatur von 175°C mittels außenliegender Ölheizung gebracht. Es wurde ein Frischgasstrom von 100 Normliter/h Wasserstoff eingestellt. Daraufhin wurden 22,5 ml/h Dimethyladipat kontinuierlich in Rieselfahrweise hydriert. Unter den Reaktionsbedingungen lag im Reaktor ein Molverhältnis von zu hydrierender Estergruppe zu Wasserstoff von 1 : 25 vor. Im Austrag fanden sich nach einer Einfahrzeit (Methanol-frei gerechnet) von 12 h 35 % Dimethyladipat, 10 % 6-Hydroxycapronsäuremethylester, 30 % 1,6-Hexandiol, 10 % Ester aus 6-Hydroxycapronsäure und 1,6-Hexandiol und 14 % Ester aus Adipinsäure, 1,6-Hexandiol und Methanol. Der Rest waren andere gemischte Ester und Oligomere. Von diesem Gemisch wurden Dimethyladipat, 6-Hydroxycapronsäuremethylester und 1,6-Hexandiol abdestilliert. Der verbleibende Rückstand wurde im Verhältnis 1 zu 5 mit frischem Dimethyladipat vermischt und unter den oben genannten Bedingungen am selben Katalysator nochmals hydriert. Es zeigt sich, daß der resultierende Hydrieraustrag der Zusammensetzung entspricht, wie sie ohne Zumischung des Destillationssumpfes erhalten wurde. An diesem Zustand änderte sich auch nach 5 Rückführungen nichts.

### Beispiel 2:

Wie in Beispiel 1 beschrieben wurde an einem Katalysator hydriert, der 1 % Re und 1 % Pt auf Aluminiumoxid enthielt (hergestellt durch Aufbringung von PtO₂ und Re₂O₇ auf Al₂O₃ und anschließende Reduktion im Wasserstoffstrom). Bei einer Hydriertemperatur von 163°C fanden sich im Austrag (Methanol-frei gerechnet) 1 % n-Hexanol, 2 % 1,6-Hexandiol, 10 % 6-Hydroxycapronsäuremethylester und 73 % Dimethyladipat. Der Rest bestand überwiegend aus dimeren und oligomeren gemischten Estern, überwiegend denen von 1,6-Hexandiol. Nach destillativer Abtrennung von n-Hexanol, Dimethyladipat, 6-Hydroxycapronsäuremethylester und 1,6-Hexandiol wurde der verbleibende Destillationssumpf im Verhältnis 1 : 6 mit frischem Dimethyladipat vermischt und wieder hydriert. Die Zusammensetzung des Hydrieraustrags veränderte sich praktisch nicht.

## Patentansprüche

1. Verfahren zur Herstellung von 1,6-Hexandiol und 6-Hydroxycapronsäure oder deren Estern durch katalytische Hydrierung von Adipinsäure, Adipinsäuremonoestern oder Adipinsäurediestern oder Eduktströmen, die Adipinsäure oder deren Ester als wesentliche Bestandteile enthalten, **dadurch gekennzeichnet, daß** man das bei der Destillation des Hydrieraustrags nach Abtrennung des Hexandiols und der Hydroxycapronsäure bzw. deren Ester erhaltene Sumpfprodukt, das im wesentlichen oligomere Ester der 6-Hydroxycapronsäure enthält, in die Hydrierung zurückführt und das so erhaltene Gemisch aus Edukt und Rückführstrom bei Temperaturen von 100 bis 300°C und Drücken von 10 bis 300 bar in der Flüssigphase und einem Molverhältnis von zu hydrierenden Carboxylgruppen zu Wasserstoff im Reaktor von 1 : 5 bis 1 : 100 an Hydrierkatalysatoren umsetzt.

2. Verfahren gemäß Anspruch 1, **dadurch gekennzeichnet, daß** man die Hydrierung an einem Festbett-Katalysator ausführt.

3. Verfahren gemäß Anspruch 1, **dadurch gekennzeichnet, daß** man die Hydrierung an Hydrierkatalysatoren durchführt, die als hydrieraktive Bestandteile ein oder mehrere Elemente der Gruppen Ib, VIb, VIIb und VIIIb, sowie IIIa, IVa und Va des Periodensystems der Elemente enthalten.

4. Verfahren gemäß Anspruch 1, **dadurch gekennzeichnet; daß** man die Hydrierung an Hydrierkatalysatoren durchführt, die als hydrieraktive Bestandteile eines oder mehrere Elemente ausgewählt aus der Gruppe bestehend aus Kupfer, Chrom, Rhenium, Kobalt, Rhodium, Nickel, Palladium, Eisen, Platin, Indium, Zinn und Antimon enthalten.

5. Verfahren gemäß Anspruch 4, **dadurch gekennzeichnet, daß** man die Hydrierung mit Trägerkatalysatoren durchführt.

6. Verfahren gemäß Anspruch 1, **dadurch gekennzeichnet, daß** man einen Hydrierkatalysator verwendet, der zumindest Kupfer, Kobalt oder Rhenium enthält.

7. Verfahren gemäß Anspruch 1, **dadurch gekennzeichnet, daß** man absatzweise arbeitet, die gesamte Menge des Destillationssumpfes des Hydrieraustrags mehrfach zurückführt und erst im Falle einer Aufpegelung von Nebenprodukten eine Ausschleusung eines Teils des Sumpfes vornimmt.

8. Verfahren gemäß Anspruch 1, **dadurch gekennzeichnet, daß** man kontinuierlich arbeitet, zumindest den überwiegenden Teil des Destillationssumpfes kontinuierlich zurückführt und gegebenenfalls einen geringeren Teil des Sumpfes kontinuierlich ausschleust.

9. Verfahren gemäß Anspruch 1, **dadurch gekennzeichnet, daß** man durch Einstellung des Wasserstoff-Überschusses, der Verweilzeit und gegebenenfalls weiterer Reaktionsparameter ein Molverhältnis von 1,6-Hexandiol zu 6-Hydroxycapronsäure bzw. deren Ester im Hydrierprodukt zwischen 1 zu 5 bis 20 zu 1 einstellt.

## Claims

1. A process for the preparation of 1,6-hexanediol and 6-hydroxycaproic acid or esters thereof by catalytic hydrogenation of adipic acid, adipic acid monoesters or adipic acid diesters or streams of starting material which contain adipic acid or esters thereof as essential constituents, which comprises recycling the bottom product which is obtained in the distillation of the hydrogenation product, following removal of the hexanediol and hydroxycaproic acid or esters thereof, and essentially comprises oligomeric esters of 6-hydroxycaproic acid, to the hydrogenation, and reacting the resulting mixture of starting material and recycle stream at from 100 to 300°C and at from 10 to 300 bar in the liquid phase and at a molar ratio of carboxyl groups to be hydrogenated to hydrogen in the reactor of from 1 : 5 to 1 : 100 on hydrogenation catalysts.

2. A process as claimed in claim 1, which comprises carrying out the hydrogenation on a fixed bed catalyst.

3. A process as claimed in claim 1, which comprises carrying out the hydrogenation on hydrogenation catalysts which comprise, as hydrogenation-active constituents, one or more elements of groups Ib, VIb, VIIb and VIIIb, and IIIa, IVa and Va, of the Periodic Table of the Elements.

4. A process as claimed in claim 1, which comprises carrying out the hydrogenation on hydrogenation catalysts which comprise, as hydrogenation-active constituents, one or more elements selected from the group consisting of copper, chromium, rhenium, cobalt, rhodium, nickel, palladium, iron, platinum, indium, tin and antimony.

5. A process as claimed in claim 4, which comprises carrying out the hydrogenation using supported catalysts.

6. A process as claimed in claim 1, which comprises using a hydrogenation catalyst containing at least copper, cobalt or rhenium.

7. A batch process as claimed in claim 1, in which all of the distillation bottom product of the hydrogenation product is repeatedly recycled, and only when there is an increase in the level of byproducts is some of the bottom product discharged.

8. A continuous process as claimed in claim 1, in which at least most of the distillation bottom product is continuously recycled and, as necessary, a relatively small amount of the bottom product is continuously discharged.

9. A process as claimed in claim 1, wherein, by adjusting the hydrogen excess, the residence time and, if necessary, other reaction parameters, a molar ratio of 1,6-hexanediol to 6-hydroxycaproic acid or esters thereof in the hydrogenation product between 1 : 5 to 20 : 1 is established.

## Revendications

1. Procédé de préparation de 1,6-hexanediol et d'acide 6-hydroxycapronique ou de ses esters par hydrogénation catalytique d'acide adipique, de monoesters de l'acide adipique ou de diesters de l'acide adipique ou de courants de charge contenant de l'acide adipique ou ses esters en tant que constituants principaux, **caractérisé en ce que** l'on renvoie à l'hydrogénation le produit de fond, obtenu lors de la distillation du produit de l'hydrogénation après séparation de l'hexanediol et de l'acide hydroxycapronique ou de son ester, produit de fond contenant pour l'essentiel des esters oligomères de l'acide 6-hydroxycapronique, et **en ce qu'**on fait réagir sur des catalyseurs d'hydrogénation le mélange ainsi obtenu du produit de charge et du courant de recyclage, à des températures de 100 à 300°C et sous des pressions de 10 à 300 bar, en phase liquide et selon un rapport en moles des groupes carboxyle à hydrogéner à l'hydrogène dans le réacteur de 1:5 à 1:100.

2. Procédé selon la revendication 1, **caractérisé en ce qu'**on met en oeuvre l'hydrogénation sur un catalyseur à lit fixe.

3. Procédé selon la revendication 1, **caractérisé en ce qu'**on met en oeuvre l'hydrogénation sur des catalyseurs d'hydrogénation qui en tant que constituants à activité d'hydrogénation contiennent un ou plusieurs éléments des groupes Ib, VIb, VIIb et VIIIb, ainsi que IIIa, IVa et Va, du Tableau Périodique des Eléments.

4. Procédé selon la revendication 1, **caractérisé en ce qu'**on met en oeuvre l'hydrogénation sur des catalyseurs d'hydrogénation qui en tant que constituants à activité d'hydrogénation contiennent un ou plusieurs éléments choisis dans l'ensemble comprenant le cuivre, le chrome, le rhénium, le cobalt, le rhodium, le nickel, le palladium, le fer, le platine, l'indium, l'étain et l'antimoine.

5. Procédé selon la revendication 4, **caractérisé en ce qu'**on met en oeuvre l'hydrogénation avec des catalyseurs supportés.

6. Procédé selon la revendication 1, **caractérisé en ce qu'**on utilise un catalyseur d'hydrogénation qui contient au moins du cuivre, du cobalt ou du rhénium.

7. Procédé selon la revendication 1, **caractérisé en ce qu'**on travaille en mode discontinu, on renvoie à plusieurs reprises en recyclage la totalité du fond de distillation du produit de l'hydrogénation, et ce n'est que dans le cas d'une augmentation de la quantité des sous-produits que l'on procède à une évacuation d'une partie du fond.

8. Procédé selon la revendication 1, **caractérisé en ce qu'**on travaille en continu, on renvoie en recyclage en continu au moins la plus grande partie du fond de distillation, et éventuellement on évacue en continu une partie plus petite du fond.

9. Procédé selon la revendication 1, **caractérisé en ce que**, par ajustement de l'excès d'hydrogène, du temps de séjour et éventuellement d'autres paramètres de la réaction, on ajuste un rapport en moles du 1,6-hexanediol à l'acide 6-hydroxycapronique ou ses esters dans le produit d'hydrogénation à une valeur comprise entre 1:5 et 20:1.
